# EUROPÄISCHE PATENTANMELDUNG

(11) **EP 4 151 713 A1**
(43) Veröffentlichungstag der Anmeldung: **22.03.2023**
(21) Anmeldenummer: 22195694.9
(22) Anmeldetag: 14.09.2022
(51) Int. Cl.: C12M 1/22, C12M 1/36, G01N 21/95, G01N 35/00, C12M 1/24, C12M 1/34, G01N 21/958, G01N 21/94, C12Q 1/22

(54) **VERFAHREN FÜR EINE AUTOMATISCHE INSPEKTION VON EINER VIELZAHL VON PLATTENARTIGEN KUNSTSTOFFTRÄGERN**

(30) Priorität: 21.09.2021 DE 102021004734
(71) Anmelder: VMT Vision Machine Technic Bildverarbeitungssysteme GmbH, 68219 Mannheim (DE)
(72) Erfinder: Gehlen, Stefan Dr., 68549 Ilvesheim (DE); Grünewald, Frank Dr., 76726 Germersheim (DE)
(74) Vertreter: Koch Müller Patentanwaltsgesellschaft mbH

(57) **Zusammenfassung**

Verfahren für eine automatische Inspektion von einer Vielzahl von plattenartigen Kunststoffträgern (KT), wobei jeder Kunststoffträger mit einem Deckel (D) verschlossen ist und einen eindeutigen Identifikationskode und ein Nährmedium aufweist, und ein Vorratsbehälter (VB) mit einer Vielzahl von Kunststoffträgern vorgesehen ist, und eine computergesteuerte Handhabungseinheit mit einem optischen Inspektionssystem (BI) vorgesehen ist, und bei der Durchführung der automatischen Inspektion in einer Inspektionsroutine wenigstens die nachfolgenden Verfahrensschritte ausgeführt werden, und mittels der Handhabungseinheit aus dem Vorratsbehälter ein Kunststoffträger entnommen und anschließend der Deckel des Kunststoffträgers entfernt wird, und der Kunststoffträger dem Inspektionssystem zugeführt wird, und in einem weiteren Verfahrensschritt der Identifikationskode gelesen wird, und in einem Verfahrensschritt mindestens ein Bild des Kunststoffträgers mit der Oberfläche der Nährlösung aufgenommen wird, und in einem Auswerteschritt das mindestens eine Bild auf ein Wachstum von Keimen und / oder Fehler in dem Kunststoffträger und das Ergebnis der Bewertung für jeden Kunststoffträger abgespeichert wird, und die Inspektionsroutine mehrfach ausgeführt wird.

## Beschreibung

Die Erfindung betrifft ein Verfahren für eine automatische Inspektion von einer Vielzahl von plattenartigen Kunststoffträgern.

Eine Inspektion von plattenartigen Kunststoffträgern, auch als Petrischalen bezeichnet, wird insbesondere in Bereich des Umwelt- und Hygienemonitorings verwendet. Insbesondere in dem Bereich der Lebensmittelindustrie, der pharmazeutischen Industrie und der medizintechnischen Industrie ist die Sicherstellung der Sauberkeit bzw. der Keimfreiheit eine wesentliche Voraussetzung für die Produktion.

Aus der US 2003 0044321 A1, der EP 2 482 079 B1, der DE 10 2015 210 842 B3, der EP 2 807 484 B1, der DE 10 2018 000437 A1 und der WO 2021 / 0081647 A1 sind Vorrichtung und Verfahren zur Lagerung und Handhabung von Kunststoffträgern bekannt.

Die jeweiligen Kunststoffträger sind im Allgemeinen als Schalen ausgebildet und weisen einen Deckel auf. Die geöffneten Schalen werden mit dem zu untersuchenden Medium in Kontakt gebracht und anschließend wieder verschlossen. Nach einer Lagerung bei einer vorgegebenen Temperatur und Zeit, um ein Wachstum von etwaigen aufgenommenen Keimen zu ermöglichen, werden die Kunststoffträger anschließend manuell von geschultem Personal begutachtet.

Sofern ein Keimwachstum oder ein sonstiger Fehler des Mediums oder der Kunststoffträger detektiert wird, ist das Ergebnis der Begutachtung positiv, anderenfalls negativ.

Es ist wesentlich alle Schalen zuverlässig mit der gleichen Qualität zu begutachten, wobei wegen einer Rückverfolgung der Kunststoffträger eine eindeutige Kennung der jeweiligen Kunststoffträger wichtig ist. Unter anderem ist es nachteilig, dass die Ergebnisse der Begutachtung je nach Personal unterschiedlich und nicht ausreichend fehlerfrei sind und die Begutachtung insgesamt sehr zeitaufwändig und kostenintensiv ist.

Neben der visuellen Begutachtung der Schalen ist auch die Dokumentation der Ergebnisse zeitaufwändig und kostenintensiv. Für die Dokumentation sind üblicherweise prozessseitige oder gar behördliche Auflagen zu beachten, beispielsweise für den Umfang, den Aufbewahrungszeitraum und/oder die Datenintegrität der Untersuchungsergebnisse.

Gerade im Bereich des betrieblichen Umwelt- und Hygienemonitorings ist der weit überwiegende Teil der inspizierten Kunststoffträger negativ, d.h. es lässt sich insbesondere kein Keimwachstum festzustellen.

Bei einem positiven Testergebnis sind üblicherweise weitere Maßnahmen notwendig. Hierbei werden die Kunststoffträger nochmals inspiziert und mittels weiterer Analysen insbesondere eine Bestimmung und Typisierung aller vorhandenen Keime oder Mikroorganismen durchgeführt. Es ist wünschenswert, dass möglichst sämtliche Erkennungsergebnisse eines automatischen Inspektionssystems so aufbereitet und dokumentiert werden, so dass die weiterführenden Untersuchungen der Kunststoffträger vereinfacht werden und so weitere Kosten eingespart werden.

Vor diesem Hintergrund besteht die Aufgabe der Erfindung darin, ein Verfahren anzugeben, das den Stand der Technik weiterbildet.

Die Aufgabe wird durch ein Verfahren für eine automatische Inspektion von einer Vielzahl von plattenartigen Kunststoffträgern mit den Merkmalen des Patentanspruchs 1 gelöst. Vorteilhafte Ausgestaltungen der Erfindung sind Gegenstand von Unteransprüchen.

Gemäß dem Gegenstand der Erfindung wird ein Verfahren für eine automatische Inspektion, d.h. für die Durchführung einer Inspektionsroutine von einer Vielzahl von plattenartigen Kunststoffträgern bereitgestellt.

Jeder Kunststoffträger ist mit einem Deckel verschlossen und weist einen eindeutigen Identifikationskode auf.

Ferner weist der Kunststoffträger ein Nährmedium auf.

Des Weiteren ist ein Vorratsbehälter mit einer Vielzahl von Kunststoffträgern vorgesehen.

Ferner ist eine computergesteuerte Handhabungseinheit mit einem optischen Inspektionssystem vorgesehen.

Bei der Durchführung der automatischen Inspektion in einer Inspektionsroutine werden wenigstens die nachfolgenden Verfahrensschritte ausgeführt.

Mittels der Handhabungseinheit wird aus dem Vorratsbehälter ein Kunststoffträger entnommen.

In einem Verfahrensschritt wird der Identifikationskode des Kunststoffträgers gelesen.

Es sei angemerkt, dass die Reihenfolge der beiden Verfahrensschritte nicht festgelegt ist.

In einer ersten Ausführungsform wird, während die Kunststoffträger noch in dem Vorratsbehälter angeordnet sind, der Identifikationskode wenigstens eines der Kunststoffträgers gelesen und abgespeichert.

In dem nachfolgenden Verfahrensschritt wird aus dem Vorratsbehälter ein mit dem Deckel verschlossener Kunststoffträger mittels der Handlingseinheit entnommen, wobei von dem entnommenen Kunststoffträger aus dem vorhergegangenen Verfahrensschritt der Identifikationskode bekannt ist.

In einer Weiterbildung weist der Vorratsbehälter an der Oberseite eine Öffnung oder einen transparenten Bereich auf. Hierdurch lässt sich der Identifikationskode besonders einfach und vorteilhaft lesen, bevor mittels der Handlingseinheit der Kunststoffträger entnommen wird.

Es versteht sich, dass in einer anderen Weiterbildung der Identifikationskode mittels einer stationären Leseeinheit erfasst. Vorzugsweise ist die Leseeinheit unmittelbar oberhalb des Vorratsbehälters angeordnet. Ein Vorteil ist es, dass sich durch die Bestimmung des Identifikationskodes der Kunststoffträger in dem Vorratsbehälter sich die gesamte Zykluszeit verringern lässt. Unter der Zykluszeit wird vorliegend die Zeitspanne zwischen zwei unmittelbar aufeinanderfolgenden Entnahmen von Kunststoffträgen aus dem Vorratsbehälter bezeichnet.

In einer zweiten Ausführungsform wird aus dem Vorratsbehälter mittels der Handlingseinheit ein mit dem Deckel verschlossener Kunststoffträger entnommen und in dem nachfolgenden Verfahrensschritt der Identifikationskode des Kunststoffträgers gelesen und abgespeichert.

In einem nachfolgenden Verfahrensschritt wird der Deckel des Kunststoffträgers entfernt.

Anschließend, d.h. nach dem Entfernen des Deckels, wird der Kunststoffträger dem Inspektionssystem zugeführt.

Es sei angemerkt, dass das Lesen des Identifikationskodes auch nach der Entfernung des Deckels bzw. auch vom Inspektionssystem selbst geleistet werden kann.

In einem Verfahrensschritt wird mindestens ein Bild des Kunststoffträgers mit der Oberfläche der Nährlösung aufgenommen.

In einem nachfolgendem Auswerteschritt werden das mindestens eine Bild auf ein Wachstum von Keimen und / oder Fehler in dem Kunststoffträger und / oder auf Fehler in der Oberfläche des Nährmediums und / oder auf Beschädigungen des Kunststoffträgers und / oder auf eine nichtzulässige Beschriftung der Kunststoffträger bewertet.

Anschließend wird das Ergebnis der Bewertung für jeden Kunststoffträger, unter Zuordnung zu dem jeweiligen Identifikationskode, abgespeichert.

Nachfolgend wird die Inspektionsroutine mehrfach ausgeführt. Anders ausgedrückt, in einem nachfolgenden Verfahrensschritt wird mittels der Handhabungseinheit ein weiterer Kunststoffträger aus dem Vorratsbehälter entnommen.

Es sei angemerkt, dass im Allgemeinen das Nährmedium meist gelartig bis fest ist. Es versteht sich jedoch, dass das Nährmedium in einer Weiterbildung auch als Flüssigkeit vorliegt.

Des Weiteren sei angemerkt, dass die plattenartigen Kunststoffträger im Allgemeinen als Schalen ausgebildet sind. Die Kunststoffträger sind vorzugsweise rund ausgeführt und werden im Allgemeinen als sogenannte Petrischalen bezeichnet.

Es versteht sich, dass der Identifikationskode vorzugsweise als QR-Kode oder Barkode oder als Datamatrixkode ausgebildet ist. Die maschinenlesbare Kodes sind besonders für ein schnelles und sicheres Auslesen der enthalten Information durch Scanner oder Kameras ausgelegt.

In einer Weiterbildung ist der Identifikationskode in Klarschrift ausgebildet, wobei sich die Klarschrift einer automatischen Zeichenerkennung (OCR) durch Scanner und Kameras erkennen und verarbeiten lässt.

In einer anderen Weiterbildung umfasst oder besteht der Identifikationskode aus einer Kombination von Klarschrift und maschinenlesbaren Kode.

Es sei angemerkt, dass der Identifikationskode sowohl als Etikett auf den Kunststoffträger oder in einem Direktdruckverfahren auf den Kunststoffträger aufgebracht ist.

In einer Weiterbildung weisen die Kunststoffträger einen Durchmesser in einem Bereich zwischen 50 mm und 300 mm oder einen Durchmesser von 55 mm und 90 mm auf.

Auch sei angemerkt, dass insbesondere die Prüfung auf eine unzulässige Beschriftung sinnvoll ist. Als eine unzulässige Beschriftung wird insbesondere eine handschriftliche Beschriftung mit einem Filzstift verstanden. In vielen Labors bzw. bei den Anwendern werden die Kunststoffträger noch mit Hand beschriftet. Derartige Kunststoffträger können bei der automatisierten Prüfung als fehlerbehaftet gekennzeichnet werden.

Zudem sei angemerkt, dass beispielsweise die Kunststoffträger insbesondere in Form von Kunststoffschalen, die Deckel der Kunststoffschalen und das Nährmedium Fehler aufweisen können, die bei der Inspektion erkannt werden müssen.

Derartige Fehler sind insbesondere Risse, abgebrochene oder angebrochene Teilstücke vor allem in dem Bereich des Randes, sowie Verfärbungen oder Blasen. Fehler in den Kunststoffteilen erhöhen das Risiko, dass nach der Probenentnahme weitere Keime in den Kunststoffträger eindringen und das Analyseergebnis verfälschen.

Auch das Medium selbst kann beispielsweise Verunreinigungen, Blasen oder Verfärbungen aufweisen, die als Fehler erkannt werden müssen. Zudem ist es möglich, dass der Kunststoffträger nur von einem Teil von dem Mediumbenetzt ist.

Es ist bekannt, dass auch weitere Fehlermöglichkeiten bei der automatischen Inspektion beachtet werden müssen, z.B. die Bildung von Kondenswasser und -tropfen.

Ein Vorteil des Verfahrens ist es, dass eine Vielzahl von plattenartigen Kunststoffträgern mit der immer gleichbleibenden Qualität automatisiert geprüft und das Ergebnis der Prüfung automatisiert weiterverarbeitet wird oder weiterverarbeitet werden kann. Ein Eingriff eines Benutzers lässt sich wesentlich reduzieren oder ganz vermeiden. Des Weiteren wird die Fehlerquote, d.h. die Anzahl der falsch bewerteten Kunststoffträger stark reduziert und der Durchsatz erhöht.

Ein weiterer Vorteil des Verfahrens ist, dass sämtliche Bereiche der Kunststoffträger inspiziert und insbesondere auch schwer erkennbare Teile der Platte, z.B. im Randbereich, sich automatisch mit hoher Präzision und Zuverlässigkeit überprüfen lassen.

Durch den Einsatz des erfindungsgemäßen Verfahrens lassen sich unter anderem unterschiedliche Wachstumszonen auf dem Kunststoffträger erkennen. Ferner lassen sich in einem oder mehreren Bildern die Keime automatisch mittels eines Computers auswerten und die Ergebnisse durch computergestützte Verfahren separieren und einzelnen Keimen zuordnen.

In einer Ausführungsform wird nach Erkennung des mindestens einen Keims zusätzlich die Anzahl sämtlicher Keime auf einem Kunststoffträger ermittelt.

Bei der automatischen Analyse der Kunststoffträger wird bei der Gewinnung von Analyseergebnissen insbesondere die Anzahl der detektierten Keime, Eigenschaften der erkannten Kolonien, wie Größe, Farbe oder Homogenität, oder gar eine Typbestimmung der einzelnen Keime ermittelt.

In einer Weiterbildung werden für die Typbestimmung die vom Inspektionssystem erkannten Kolonien mit Referenzbildern oder die aus Bildaufnahmen der Kolonien ermittelten Merkmale mit den Merkmalen von Bildern bekannter Keimwachstumsprozessen verglichen und zugeordnet.

Der Vergleich mit Referenzen für die Erkennung des Keimwachstums in den Bildern und insbesondere für die Segmentierung und das Zählen von Kolonien sowie der Typbestimmung bzw. Zuordnung der Bilder zu Keimtypen wie unter anderem von Bakterien und Pilzen mit entsprechenden Untergruppen vereinfacht die Auswertung erheblich.

In einer Ausführungsform werden bei jedem inspizierten Kunststoffträger das Ergebnis der Bewertung und der Identifikationskode einander zugeordnet und abgespeichert.

In einer anderen Ausführungsform wird, sofern in dem Auswerteschritt ein einziger Fehler detektiert wird, der Kunststoffträger als schlecht bewertet oder sofern in dem Auswerteschritt kein Fehler detektiert wird, der Kunststoffträger als gut bewertet.

In einer Weiterbildung weist die Aufbewahrungseinheit einen Bereich für eine Lagerung von den als schlecht bewerteten Kunststoffträgern auf.

In einer Weiterbildung weist die Aufbewahrungseinheit einen Bereich für eine Lagerung von den als gut bewertenden Kunststoffträgern auf. In einer anderen Weiterbildung ist der Bereich für die Lagerung den als gut bewertenden Kunststoffträgern als Entsorgungseinheit ausgebildet.

Es versteht sich, dass jeder Verfahrensschritt oder Handhabungsschritt in einem Computer- oder Datenbanksystem für jede Kunststoffträgereinheit eindeutig nachvollziehbar abgelegt ist.

In einer Ausführungsform sind die Kunststoffträger als Sedimentationsplatten oder als Abklatschplatten ausgebildet.

In einer Weiterbildung wird ein Bild von dem Deckel aufgenommen und in dem Auswertungsschritt das Bild des Deckels auf Beschädigungen bewertet.

In einer anderen Weiterbildung werden mittels der Inspektionseinheit mehrere Bilder unter einem einzigen Betrachtungswinkel oder aus mehreren Betrachtungswinkeln aufgenommen.

In einer Ausführungsform werden für die Aufnahme der mehreren Bilder unterschiedliche Lichtquellen oder Betrachtungswinkel gewählt. Hierdurch lässt sich die Fehlerquote stark verringern.

Insbesondere ist es für eine Analyse des Nährmediums bzw. des Kunststoffträgers hilfreich, neben einer beispielsweise diffusen Beleuchtung in Auflicht, zusätzlich eine Durchlichtbeleuchtung durchzuführen. Ferner hat es sich gezeigt, dass mit speziellen Lichtquellen, wie beispielsweise UV-Quellen und mittels der Auswertung der Fluoreszenz Keime oder Fehler in den Schalen besonders einfach und zuverlässig erkennen lassen.

In einer Weiterbildung werden bei der Aufnahme von mehreren Bildern die beteiligten Kameras auf unterschiedliche Fokusebenen oder auf unterschiedliche Bereiche der Kunststoffträger eingestellt. Anders ausgedrückt für die Analyse des Nährmediums bzw. des Kunststoffträgers werden die Fokusebenen von den mehreren Bildern auf unterschiedliche Objektbereiche eingestellt. So stellt beispielsweise die Oberfläche des Nährmediums eine erste Fokusebene und die Unterseite des Kunststoffträgers eine zweite Fokusebene dar.

Unterschiedliche Fokusebenen können beispielsweise durch mehrere unterschiedlich fokussierte Kameras oder eine aktive, motorische Verstellung der Objektive einer Kamera oder eine aktive Veränderung des Abstands von Kunststoffträger und Kamera erzielt werden.

Die Auswertung von mehreren Bildern, Blickwinkeln, Fokusebenen und/oder Beleuchtungsquellen ist besonders geeignet, um ein Keimwachstum in bestimmten Bereichen der Kunststoffträger und insbesondere im Randbereich der Kunststoffträger sicher zu erkennen. Die Art von Auswertung ist aber vorteilhaft, um Fehler im Nährmedium oder Fehler in oder auf der Kunststoffschale sicher zu erkennen.

Des Weiteren hat es sich gezeigt, dass mittels der Auswertung von mehreren Bildern, Blickwinkeln, Fokusebenen und/oder Beleuchtungsquellen bestimmte Eigenschaften des Kunststoffträgers selbst, wie z.B. Laschen, Gitterstrukturen, Symbole oder im Kunststoffträger enthaltene Produktkennzeichnungen, erkannt und nicht fälschlicherweise als Fehler markiert werden.

Zur Qualitätssicherung werden in gewissen zeitlichen Abständen Bilder von Prüfkörpern bzw. Referenzkörpern aufgenommen und das Ergebnis der Auswertung der Bilder mit vorgegeben Daten verglichen. Sofern eine Differenz zwischen der Auswertung und den vorgegebenen Referenzdaten einen Grenzwert überschreitet, wird eine Diagnose oder Kalibration durchgeführt.

In einer Ausführungsform ist die Handhabungseinheit als Teil eines Roboters ausgebildet.

In einer Weiterbildung wird in einem weiteren Verfahrensschritt der Kunststoffträger wieder mit dem Deckel versehen und anschließend einer Aufbewahrungseinheit zugeführt.

In einer anderen Weiterbildung wird der Kunststoffträger mit oder ohne Deckel einer Entsorgungseinheit zugeführt. Es versteht sich, dass hierbei unter Berücksichtigung des Identifikationskodes die Entsorgung vermerkt wird.

In einer Ausführungsform werden alle Inspektionsschritte und Prüfergebnisse sowie etwaige Benutzerzugriffe in Abhängigkeit des Identifikationskodes des jeweiligen Kunststoffträgers erfasst, dokumentiert und über eine Schnittstelle bereitgestellt. Ein Vorteil ist, dass sich hierdurch insbesondere für eine Auditierung alle Informationen einfach bereitstellen lassen.

In einer Ausführungsform wird das Verfahren für ein Umwelt- oder Hygienemonitoring verwendet.

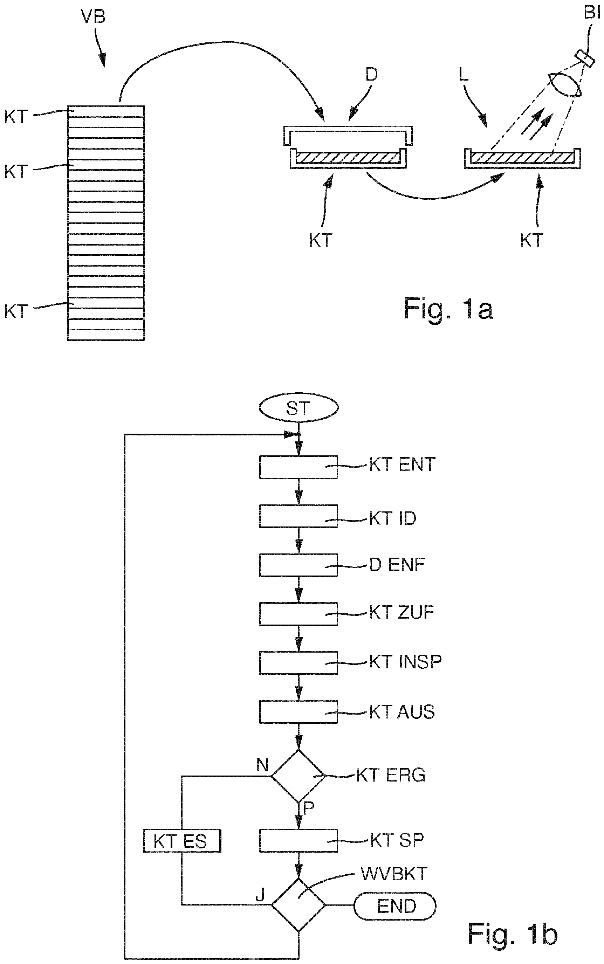

Die Erfindung wird nachfolgend unter Bezugnahme auf die Zeichnungen näher erläutert. Hierbei werden gleichartige Teile mit identischen Bezeichnungen beschriftet. Die dargestellten Ausführungsformen sind schematisiert, d.h. die Abstände und die lateralen und die vertikalen Erstreckungen sind nicht maßstäblich und weisen, sofern nicht anders angegeben, auch keine ableitbaren geometrischen Relationen zueinander auf. Darin zeigen, die
- Figur 1a: eine Darstellung der optischen Inspektion,
- Figur 1b: einen Ablaufplan für eine erste Ausführungsform des erfindungsgemäße Verfahrens,

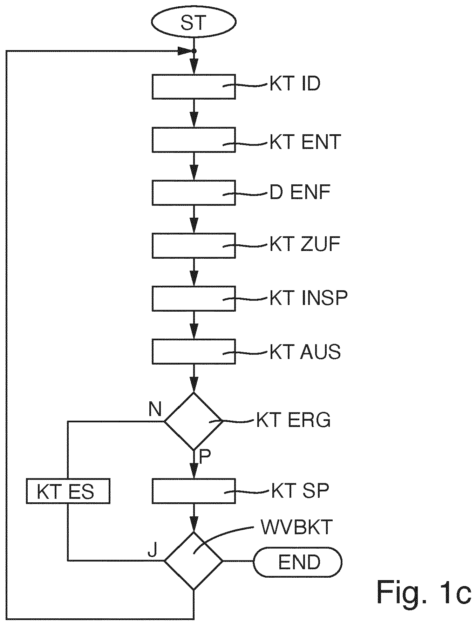

- Figur 1c: einen Ablaufplan für eine zweite Ausführungsform des erfindungsgemäßen Verfahrens.

Die Abbildung der Figur 1a zeigt eine Darstellung der optischen Inspektion mit einem Stapel aus Kunststoffträgern KT in einem Vorratsbereich VB.

Mittels einer nicht dargestellten Handlingseinheit wird von dem Stapel der oberste mit einem Deckel verschlossene Kunststoffträger KT aufgegriffen und in einem Teil der Handlingseinheit der Deckel D des Kunststoffträgers entfernt. Mittels des Lichtes L einer Lichtquelle wird der Kunststoffträger KT optisch inspiziert, indem mittels der Kameraeinheit BI wenigstens ein Bild aufgenommen wird. In einer nicht dargestellten Weiterbildung wird auch der Deckel D optisch inspiziert.

In der Figur 1b ist ein Ablaufplan für eine erste Ausführungsform des Verfahrens dargestellt, wobei nur die Unterschiede zu den in Zusammenhang mit in der Fig. 1a dargestellten Ausführungsform erläutert werden.

In einen Verfahrensschritt KTENT wird aus dem Vorratsbehälter VB ein mit dem Deckel D verschlossener Kunststoffträger KT mittels der Handlingseinheit entnommen. In einem Verfahrensschritt KTID wird der Identifikationskode des Kunststoffträgers KT gelesen und abgespeichert. In einem nächsten Verfahrensschritt DENF wird der Deckel D entfernt.

In einem weiteren Verfahrensschritt KTZUF wird der Kunststoffträger KT in den Teil mit der optischen Inspektionseinheit abgelegt. In einem weiteren Verfahrensschritt KTINSP wird mittels des Lichtes L die Oberfläche des Kunststoffträgers beleuchtet und wenigstens mittels der Sensoreinheit BI zumindest ein Bild aufgenommen.

In einem darauffolgenden Verfahrensschritt KTAUS wird das Bild ausgewertet. Sofern in dem Auswertungsschritt KTERG das Ergebnis negativ N ist, d.h. insbesondere kein Keimwachstum oder Pilzwachstum detektiert wird, wird in einem anschließenden Verfahrensschritt KTES der Kunststoffträger KT einer Entsorgungseinheit zugeführt.

Sofern in dem Auswertungsschritt KTERG das Ergebnis positiv P ist, d.h. insbesondere ein Keimwachstum oder Pilzwachstum detektiert wird, wird in einem anschließenden Verfahrensschritt KTSP der Kunststoffträger KT einer Aufbewahrungseinheit zugeführt. Im Auswerteschritt KTERG werden in einer Weiterbildung weitere Analyseschritte beispielweise hinsichtlich der Ermittlung der Keimzahl und der Art der Keime durchgeführt.

Sowohl nach dem Verfahrensschritt KTES als nach dem Verfahrensschritt KTSP wird in einem Abfrageschritt WVBKT geprüft, ob weitere Kunststoffträger KT inspiziert werden sollen.

Ist das Ergebnis der Prüfung Nein N wird das Verfahren beendet. Ist das Ergebnis der Prüfung Ja J wird das Verfahren von Beginn an wiederholt und von dem Vorratsbehälter VB der nächste Kunststoffträger KT entnommen.

In der Figur 1c ist ein Ablaufplan für eine zweite Ausführungsform des Verfahrens dargestellt, wobei nur die Unterschiede zu den in Zusammenhang mit in der Fig. 1b dargestellten Ausführungsform erläutert werden.

Während die Kunststoffträger KT noch in dem Vorratsbehälter VB angeordnet sind, wird in dem Verfahrensschritt KT ID der Identifikationskode ID wenigstens eines der Kunststoffträgers KT gelesen und abgespeichert.

In dem nachfolgenden Verfahrensschritt KT ENT wird aus dem Vorratsbehälter VB ein mit dem Deckel D verschlossener Kunststoffträger KT mittels der Handlingseinheit entnommen, wobei von dem Kunststoffträger KT aus dem vorhergegangenen Verfahrensschritt KT ID der Identifikationskode ID bekannt ist. In einem nächsten Verfahrensschritt DENT wird der Deckel D entfernt.

## Patentansprüche

1. Verfahren für eine automatische Inspektion von einer Vielzahl von plattenartigen Kunststoffträgern (KT) und jeder Kunststoffträger (KT) mit einem Deckel (D) verschlossen ist und einen eindeutigen Identifikationskode und ein Nährmedium aufweist, wobei
- ein Vorratsbehälter (VB) mit einer Vielzahl von Kunststoffträgern (KT) vorgesehen ist,
- eine computergesteuerte Handhabungseinheit mit einem optischen Inspektionssystem vorgesehen ist,
- bei der Durchführung der automatischen Inspektion in einer Inspektionsroutine wenigstens die nachfolgenden Verfahrensschritte ausgeführt werden,
- mittels der Handhabungseinheit aus dem Vorratsbehälter (VB) ein Kunststoffträger (KT) entnommen und anschließend der Deckel (D) des Kunststoffträgers (KT) entfernt wird,
- der Kunststoffträger (KT) dem Inspektionssystem zugeführt wird,
- in einem Verfahrensschritt (KTID) der Identifikationskode gelesen wird,
- in einem Verfahrensschritt mindestens ein Bild des Kunststoffträgers (KT) mit der Oberfläche der Nährlösung aufgenommen wird,
- in einem Auswerteschritt (KTINSP) das mindestens eine Bild auf ein Wachstum von Keimen und / oder Fehler in dem Kunststoffträger (KT) und / oder Fehler in der Oberfläche des Nährmediums und / oder auf Beschädigungen des Kunststoffträgers (KT) und / oder auf eine nichtzulässige Beschriftung der Kunststoffträger (KT) bewertet werden,
- das Ergebnis der Bewertung für jeden Kunststoffträger (KT) abgespeichert wird,
- die Inspektionsroutine mehrfach ausgeführt wird.

2. Verfahren für eine automatische Inspektion nach Anspruch 1, **dadurch gekennzeichnet, dass** bei jedem inspizierten Kunststoffträger (KT) das Ergebnis der Bewertung und der Identifikationskode einander zugeordnet und abgespeichert werden.

3. Verfahren für eine automatische Inspektion nach Anspruch 1 oder Anspruch 2, **dadurch gekennzeichnet, dass** sofern in dem Auswerteschritt ein einziger Fehler detektiert wird, der Kunststoffträger (KT) als schlecht bewertet oder sofern in dem Auswerteschritt kein Fehler detektiert wird, der Kunststoffträger (KT) als gut bewertet wird.

4. Verfahren für eine automatische Inspektion nach Anspruch 3, **dadurch gekennzeichnet, dass** die Aufbewahrungseinheit einen Bereich für eine Lagerung von den als schlecht bewerteten Kunststoffträgern (KT) und einen Bereich für eine Lagerung von den als gut bewertenden Kunststoffträgern (KT) aufweist.

5. Verfahren für eine automatische Inspektion nach einem der vorstehenden Ansprüche **dadurch gekennzeichnet, dass** die Kunststoffträger (KT) als Sedimentationsplatten oder als Abklatschplatten ausgebildet sind.

6. Verfahren für eine automatische Inspektion nach einem der vorstehenden Ansprüche, **dadurch gekennzeichnet, dass** ein Bild von dem Deckel (D) aufgenommen wird und in dem Auswertungsschritt das Bild des Deckels (D) auf Beschädigungen bewertet wird.

7. Verfahren für eine automatische Inspektion nach einem der vorstehenden Ansprüche, **dadurch gekennzeichnet, dass** mittels der Inspektionseinheit mehrere Bilder unter einem einzigen Betrachtungswinkel oder aus mehreren Betrachtungswinkeln aufgenommen werden.

8. Verfahren für eine automatische Inspektion nach Anspruch 7, **dadurch gekennzeichnet, dass** für die Aufnahme der mehreren Bilder unterschiedliche Beleuchtungsquellen verwendet werden.

9. Verfahren für eine automatische Inspektion nach Anspruch 7, **dadurch gekennzeichnet, dass** für die Aufnahme der mehreren Bilder mehrere Fokusebenen verwendet werden.

10. Verfahren für eine automatische Inspektion nach einem der vorstehenden Ansprüche, **dadurch gekennzeichnet, dass** zur Qualitätssicherung Bilder von Prüfkörpern aufgenommen und die Bilder anschließend ausgewertet werden und das Ergebnis der Auswertung mit vorgegeben Daten verglichen wird und sofern eine Differenz zwischen der Auswertung und den vorgegebenen Daten einen Grenzwert überschreitet, Inspektionsroutinen durchgeführt werden.

11. Verfahren für eine automatische Inspektion nach einem der vorstehenden Ansprüche, **dadurch gekennzeichnet, dass** in einem weiteren Verfahrensschritt der Kunststoffträger (KT) wieder mit dem Deckel (D) versehen und einer Aufbewahrungseinheit zugeführt wird.

12. Verfahren für eine automatische Inspektion nach einem der vorstehenden Ansprüche, **dadurch gekennzeichnet, dass** alle Inspektionsschritte und Prüfergebnisse und Benutzerzugriffe erfasst und dokumentiert werden und über eine Schnittstelle ausgebbar sind.

13. Verfahren für eine automatische Inspektion nach einem der vorstehenden Ansprüche, bei dem nach Erkennung des mindestens einen Keims zudem die Anzahl sämtlicher Keime auf einem Kunststoffträger ermittelt wird.

14. Verfahren für eine automatische Inspektion nach einem der vorstehenden Ansprüche, bei dem nach Erkennung der Keime für jeden Keim eine Typklassifizierung vorgenommen wird.

15. Verfahren für eine automatische Inspektion nach einem der vorstehenden Ansprüche, bei dem der Identifikationskode der Kunststoffträger (KT) in dem Vorratsbehälter (VB) vor dem Entnehmen der Kunststoffträger (KT) gelesen und bestimmt wird.
